# EUROPEAN PATENT APPLICATION

(11) **EP 3 002 011 A1**
(43) Date of publication of application: **06.04.2016**
(21) Application number: 14798312.6
(22) Date of filing: 06.02.2014
(51) Int. Cl.: A61K 35/12, A61K 38/30, A61K 31/375, A61K 9/08

(54) **STEM CELL COMPOSITION FOR VENOUS ADMINISTRATION**

(30) Priority: 15.05.2013 KR 20130055158
(71) Applicant: Ra, Jeong-Chan, Suwon-si, Gyeonggi-do 440-735 (KR); RBioCo., Ltd., Yeongdeungpo-gu Seoul 150-870 (KR)
(72) Inventor: KANG, Sung Keun, Seoul 151-770 (KR); JO, Jung Youn, Seoul 138-859 (KR)
(74) Representative: von Kreisler Selting Werner - Partnerschaft von Patentanwälten und Rechtsanwälten mbB
(86) International application number: PCT/KR2014/001012
(87) International publication number: WO 2014/185620

(57) **Abstract**

The present invention relates to a stem cell composition for venous administration, the composition containing between 1x10⁷ and 5x10⁸ cells/ml of stem cells present as single cells and having a diameter of between 10 and 20 µm. The present invention makes it possible to provide a stem cell composition suitable for venous administration, and makes it possible to relatively efficiently enhance the efficacy with which vascularly administered stem cells stably reach target tissue and exhibit an action, and can therefore dramatically increase the efficacy of cell therapy using stem cells.

## Description

### TECHNICAL FIELD

The present invention relates to a stem cell composition for intravenous administration, and more particularly, to a stem cell composition for intravenous administration, which contains stem cells at a concentration of 1 X 10⁷ to 5 X 10⁸ cells/ml together with an excipient, in which the stem cells have a diameter suitable for intravascular administration and are present as single cells.

### BACKGROUND ART

Stem cells refer to cells having not only self-replicating ability but also the ability to differentiate into at least two types of cells, and can be divided into totipotent stem cells, pluripotent stem cells, and multipotent stem cells. Totipotent stem cells are cells having totipotent properties capable of developing into one perfect individual, and these properties are possessed by cells up to the 8-cell stage after the fertilization of an oocyte and a sperm. When these cells are isolated and transplanted into the uterus, they can develop into one perfect individual. Pluripotent stem cells, which are cells capable of developing into various cells and tissues derived from the ectodermal, mesodermal and endodermal layers, are derived from an inner cell mass located inside of blastocysts generated 4-5 days after fertilization. These cells are called "embryonic stem cells" and can differentiate into various other tissue cells but not form new living organisms. Multipotent stem cells, which are stem cells capable of differentiating into only cells specific to tissues and organs containing these cells, are involved not only in the growth and development of various tissues and organs in the fetal, neonatal and adult periods but also in the maintenance of homeostasis of adult tissue and the function of inducing regeneration upon tissue damage. Tissue-specific multipotent cells are collectively called "adult stem cells".

Adult stem cells are obtained by taking cells from various human organs and developing the cells into stem cells and are characterized in that they differentiate into only specific tissues. However, recently, experiments for differentiating adult stem cells into various tissues, including liver cells, were dramatically successful, which comes into spotlight. In particular, efforts have been made in the field of regenerative medicine for regenerating biological tissues and organs and recovering their functions that were lost due to illness or accident and the like by using cells. Methods which are frequently used in this field of regenerative medicine comprise the steps of: collecting stem cells, blood-derived mononuclear cells or marrow-derived mononuclear cells from a patient; inducing the proliferation and/or differentiation of the cells by tube culture; and introducing the selected undifferentiated (stem cells and/or progenitor cells) and/or differentiated cells into the patient's body by transplantation. Accordingly, existing classical methods for treating diseases by medication or surgery are expected to be replaced with cell/tissue replacement therapy which replaces a damage cell, tissue or organ with healthy one, and thus the utility of stem cells will further increase.

Thus, the various functions of stem cells are currently being studied. Particularly, since cell therapy technologies using mesenchymal stem cells started to receive attention, technologies for improving mesenchymal stem cells isolated from a human body so as to be suitable for therapeutic purposes have been developed (WO 2006/019357, Korean Patent No. 0795708, and Korean Patent No. 0818214, Leu, Steve Lin et al., Journal of translational medicine, 8(1):63, 2010; Kim, J.M. et al., Brain research, 1183:43, 2007, Kim, Young-Ki et al., Journal of veterinary clicics, 28:122, 2011; Park SS. et al., Cytotherapy, 14(5):584, 2012; Soo-Kyung Kang et al., Stem Cells and Development, 15(4):583, 2006). Stem cells can be administered not only directly into injury sites having arthritis or degenerative arthritis to exhibit their therapeutic effects, but also into veins. Stem cells that are administered intravenously can be delivered directly into injured body sites such as injured organs to exhibit the effect of treating the injured sites, and thus are expected to exhibit therapeutic effects against various diseases, including neurological diseases, Alzheimer's disease, cancer, diabetes, and rheumatoid arthritis.

However, a technology related to methods for preparing stem cells compositions suitable for intravascular administration has not yet been sufficiently studied.

Accordingly, the present inventors have made extensive efforts to develop a stem cell composition for intravenous administration, which can securely reach a target site so as to exhibit their therapeutic effects in the target site, and as a result, have found a stem cell composition for intravenous administration, which contains stem cells which are neither disrupted nor form aggregates before intravenous administration, have a diameter suitable for intravenous administration, are present as single cells, and are contained at a concentration suitable for exhibiting their therapeutic effects, thereby completing the present invention.

### DISCLOSURE OF INVENTION

It is an object of the present invention to provide a stem cell composition for intravenous administration.

To achieve the above object, the present invention provides a stem cell composition for intravenous administration, which contains stem cells at a concentration of 1 X 10⁷ to 5 X 10⁸ cells/ml, in which the stem cells have a diameter of 10-20 *µ*m and are present as single cells.

Other features and embodiments of the present invention will be more apparent from the following detailed descriptions and the appended claims.

### BEST MODE FOR CARRYING OUT THE INVENTION

Unless defined otherwise, all technical and scientific terms used herein have the same meaning as commonly understood by one of ordinary skill in the art to which the invention pertains. Generally, the nomenclature used herein are well known and are commonly employed in the art.

As used herein, the term "stem cells" refer to cells having not only self-replicating ability but also the ability to differentiate into at least two types of cells. "Adult stem cells" refer to stem cells that appear either in the stage in which each organ of an embryo is formed after the developmental process or in the adult stage.

As used herein, the term "mesenchymal stem cells" refers to undifferentiated stem cells that are isolated from human or mammalian tissue and may be derived from various tissues. Particularly, the mesenchymal stem cells may be umbilical cord-derived mesenchymal stem cells, umbilical cord blood-derived mesenchymal stem cells, bone marrow-derived mesenchymal stem cells, adipose-derived mesenchymal stem cells, muscle-derived mesenchymal stem cells, nerve-derived mesenchymal stem cells, skin-derived mesenchymal stem cells, amnion-derived mesenchymal stem cells, or placenta-derived mesenchymal stem cells. Technology for isolating stem cells from each tissue is already known in the art.

As used herein, "adipose tissue-derived mesenchymal stem cells" are undifferentiated adult stem cells isolated from adipose tissue and are also referred to herein as "adipose-derived adult stem cells", "adipose stem cells", or "adipose-derived stem cells". These cells can be obtained according to any conventional method known in the art. A method for isolating adipose tissue-derived mesenchymal stem cells may be, for example, as follows. That is, adipose-derived mesenchymal stem cells can be isolated by culturing an adipose-containing suspension (in physiological saline) obtained by liposuction, and then either collecting a stem cell layer, attached to a culture container such as a flask, by trypsin treatment, or directly collecting those suspended in a small amount of physiological saline by rubbing with a scraper.

As used herein, the expression "stem cells having a size suitable for intravascular administration" refers to stem cells that preferably have a diameter of 10-20 *µ*m, and more preferably 10-15 *µ*m, which is smaller than the diameter of veins, so as to be able to easily migrate into a target tissue without interfering with blood flow or circulation after intravenous administration to thereby to exhibit their activity in the target tissue.

As used herein, the term "transport" means transporting stem cells themselves, a container containing a solution containing stem cells, or the like, by transportation means such as vehicles, and the term "storage" is intended to include not only storage at room temperature, but also storage under cold conditions.

As used herein, the expression "preventing the disruption and aggregation of stem cells" means maintaining the shape of stem cells as single cells without disruption or aggregation. For example, it means that stem cells are maintained as single cells without disruption of the cell membrane or aggregation of the cells during transport or storage.

Stem cells can be administered into the body by various routes, for example, an intravenous, intraarterial or intraperitoneal route. Among these administration routes, intravenous administration is useful in that it can simply and safely treat a disease without a surgical operation. However, in order for stem cells to securely reach a target site after intravenous administration so as to exhibit their therapeutic effects in the target site, various requirements should be satisfied.

First, stem cells should have a size suitable for intravascular administration so as not to reduce blood flow rate or to form thrombi after intravascular administration. Mesenchymal stem cells that can be isolated from various tissues have different morphologies or proliferate to different degrees, depending on patients, their origin, or culture conditions or methods, and also have various sizes ranging from about 10 *µ*m to 300 *µ*m. However, human post-capillary venules have a diameter of 20-50 *µ*m, human arterioles have a diameter of 10-30 *µ*m (Schmidt GT, 1989), and capillary vessels have a diameter of about 10 *µ*m (Schmidt GT, 1989; Chien, 1975; John Ross, 1991; Herbert *et al., 1989; Arthur and Guyton,* 1997; Renkin, 1989; Gaehtgens, 1980; Row 1979). These diameters are smaller than the diameter of common mesenchymal stem cells. For this reason, when mesenchymal stem cells having a relatively large size are administered intravenously, they can affect blood vessels. Specifically, the intravenously administered mesenchymal cells can reduce blood flow rate and also interfere with blood circulation to cause blood flow interruption, thrombus formation, vascular occlusion, and even death. Regarding these side effects, it was reported that, when mesenchymal stem cells having a diameter of about 20-53 *µ*m were administered intravenously to mice, blood flow rate was reduced and the induction of myocardial infarction and thrombus formation was observed (D.Furlani et al. Microvasular Research 77 (2009) 370-376). Thus, it is important to administer stem cells having a proper size into blood vessels.

In addition, stem cells should not be disrupted or should not aggregate, before intravascular administration, and should be maintained as single cells and should securely reach a target site without being disrupted or forming aggregates, after intravascular administration. Stem cells can be treated with trypsin to form single cells for administration into the body, but even stem cells prepared as single cells have a problem in that the cell membrane is disrupted or the cells form aggregates, during transport or storage. When either aggregated stem cells that are not single cells, or disrupted cells, are administered into the body by a route such as intravenous administration, these administered cells can adhere to vascular endothelial cells or platelets to reduce blood flow rate or interfere with blood circulation, and even cause occlusion of blood microvessels or blood vessels (D.Furlani et al. Microvasular Research 77 (2009) 370-376). Thus, stem cells should not be disrupted or should not aggregate, before intravascular administration, and should be maintained as single cells and should securely reach a target site without being disrupted or forming aggregates, after intravascular administration.

In addition, a certain concentration or higher of stem cells should be administered so that the stem cells that reached a target site will exhibit their therapeutic effects in the target site. Thus, it is important to prepare a large amount of the stem cells to be clinically applied.

Therefore, the present invention is to indented to provide a highly safe stem cell composition for intravenous administration, which contains stem cells and prevents the disruption and aggregation of the stem cells before intravascular administration to make the stem cells suitable for administration into the body, in which the stem cells have a size suitable for intravascular administration so as not to reduce blood flow rate or form thrombi after intravascular administration, and thus surely exhibit their therapeutic effects.

In one aspect, the present invention is directed to a stem cell composition for intravenous administration, which contains stem cells at a concentration of 1 X 10⁷ to 5 X 10⁸ cells/ml, in which the stem cells have a diameter of 10-20 *µ*m and are present as single cells.

As stem cells used in the present invention, adult stem cells derived from adipose tissue of the adult stem cells or epithelial tissue such as a hair follicle or an amnion may be used. Preferably, mesenchymal stem cells (MSCs) are used. Most preferably, human adipose tissue-derived mesenchymal stem cells (AdMSCs) may be used.

Said adipose tissue or epithelial tissue is preferably derived from a mammal, more preferably a human. In one examples of the present invention, human adipose tissue-derived mesenchymal stem cells (AdMSCs) were used.

In the present invention, the stem cells contained in the stem cell composition for intravenous administration are prepared by the culture thereof in a medium containing a basal medium; and at least two components selected from the group consisting of N-acetyl-L-cysteine (NAC), ascorbic acid, insulin or insulin-like factor, hydrocortisone, dexamethasone, bFGF (basic fibroblast growth factor), heparan sulfate, 2-mercaptoethanol, EGF (epidermal growth factor), and antioxidant.

A basal medium that is used in a medium for preparing the stem cells to be contained in the stem cell composition of the present invention is a conventional medium having a simple composition, which is known in the art to be suitable for the culture of stem cells. Examples of the basal medium generally used to culture the stem cells include MEM (Minimal Essential Medium), DMEM (Dulbecco modified Eagle Medium), RPMI (Roswell Park Memorial Institute Medium), and K-SFM (Keratinocyte Serum Free Medium). As the basal medium used in the present invention, any mediums can be used without any limitation as long as they are used in the art. Preferably, the basal medium may be selected from the group consisting of M199/F12(mixture)(GIBCO), MEM-alpha medium (GIBCO), low-concentration glucose-containing DMEM medium (Welgene), MCDB 131 medium (Welgene), IMEM medium(GIBCO), K-SFM, DMEM/F12 medium, PCM medium, and MSC expansion medium (Chemicon). Particularly, among them, K-SFM may be preferably used.

A basal medium that is used to obtain the cultured mesenchymal stem cells may be supplemented with additives known in the art, which promote the proliferation of mesenchymal stem cells in an undifferentiated state while inhibiting the differentiation thereof. Also, the medium may contain a neutral buffer (such as phosphate and/or high-concentration bicarbonate) in isotonic solution, and a protein nutrient (e.g., serum such as FBS, FCS (fetal calf serum) or horse serum, serum replacement, albumin, or essential or non-essential amino acid such as glutamine or L-glutamine). Furthermore, it may contain lipids (fatty acids, cholesterol, an HDL or LDL extract of serum) and other ingredients found in most stock media of this kind (such as insulin or transferrin, nucleosides or nucleotides, pyruvate, a sugar source such as glucose, selenium in any ionized form or salt, a glucocorticoid such as hydrocortisone and/or a reducing agent such as β-mercaptoethanol).

Also, for the purpose of preventing cells from adhering to each other, adhering to a vessel wall, or forming too large clusters, the medium may advantageously contain an anti-clumping agent, such as one sold by Invitrogen (Cat # 0010057AE).

Among them, one or more of the following additional additives may advantageously be used:
- stem cell factor (SCF, Steel factor), other ligands or antibodies that dimerize c-kit, and other activators of the same signaling pathway
- ligands for other tyrosine kinase related receptors, such as the receptor for platelet-derived growth factor (PDGF), macrophage colony-stimulating factor, Flt-3 ligand and vascular endothelial growth factor (VEGF)
- factors that elevate cyclic AMP levels, such as forskolin
- factors that induce gp130 such as LIF or Oncostatin-M
- hematopoietic growth factors such as thrombopoietin (TPO)
- transforming growth factors such as TGFβ1
- neurotrophins such as CNTF
- antibiotics such as gentamicin, penicillin or streptomycin.

The medium for preparing the stem cells to be contained in the stem cell composition of the present invention may contain, in addition to the basal medium, at least two components selected from the group consisting of N-acetyl-L-cysteine (NAC), ascorbic acid, insulin or insulin-like factor, hydrocortisone, dexamethasone, bFGF (basic fibroblast growth factor), heparan sulfate, 2-mercaptoethanol, EGF (epidermal growth factor), and antioxidant.

Specifically, the medium may contain insulin-like factor as insulin replacement, which functions to promote cell growth by enhancing glucose metabolism and protein metabolism. Particularly, recombinant IGF-1 (insulin-like growth factor-1) is preferably used. The preferred content of insulin-like factor is 10-50 ng/ml. If the content of insulin-like factor is less than 10 ng/ml, apoptosis will occur, and if the content is more than 50 ng/ml, it will increase the cytotoxicity and cost of the medium. The content of hydrocortisone may be 60-80 ng/ml.

The medium may contain basic fibroblast growth factor (bFGF) that can induce various types of cell proliferation *in vivo.* Preferably, recombinant bFGF protein is used. The preferred content of bFGF is 1-100 ng/ml.

The stem cell composition of the present invention may contain an antioxidant. The antioxidant that is used in the stem cell composition of the present invention may be selected from among selenium, ascorbic acid, vitamin E, catechin, lycopene, beta-carotene, coenzyme Q-10, EPA (eicosapentaenoic acid), DHA (docosahexanoic acid) and the like. Preferably, the antioxidant may be selenium. In an example of the present invention, selenium was used as an antioxidant. The amount of selenium used is preferably 0.5-10 ng/ml. If the content of selenium is less than 0.5 ng/ml, the stem cell composition will be susceptible to oxygen toxicity, and if it is more than 10 ng/ml, the stem cell composition can cause serious cytotoxicity. When the stem cell composition contains ascorbic acid as an antioxidant, the content of ascorbic acid in the composition may be 0.1-0.3 mM.

The medium that is used in the present invention may additionally contain a component selected from the group consisting of FBS (fetal bovine serum), calcium and EGF. The content of calcium may be 0.05-0.13 Mm. Epidermal growth factor (EGF) may cause proliferation of cells in various forms *in vivo,* and preferably uses recombinant EGF protein. The preferred content of epidermal growth factor is 10-50 ng/ml. If the content of epidermal growth factor in the medium is less than 10 ng/ml, it will have no particular effect, and if the content is more than 50 ng/ml, it will be toxic to cells.

Stem cells cultured in the medium according to the present invention preferably have a diameter of 10-20 *µ*m, and more preferably 10-15 *µ*m, and thus are suitable for intravascular administration.

Moreover, Preferably, when subcultured 4-6 times, stem cells cultured in the medium according to the present invention can be proliferated at a concentration of 7x 10⁵ - 5 x 10⁸ cells/ml. In addition, since functional/morphological deformation of cells does not occurs during the increase in the number of the stem cells by the subculture, the stem cells obtained according to the present invention can be effectively applied to a clinical trial. In conventional methods for culturing stem cells, the stem cells should be subcultured several times in order to increase the yield of the stem cells, and thus large amounts of manpower and time are required. Particularly, in this case, some of the medium components required for subculture are very costly, and thus the conventional medium compositions are disadvantageous in economic terms. However, when the medium according to the present invention is used, it is possible to prepare a high concentration of clinically applicable stem cells by performing subculture only three to five times.

Therefore, in another aspect, the present invention provides a medium for preparing stem cells suitable for intravenous administration, the medium containing: a basal medium; and two or more selected from the group consisting of NAC (N-acetyl-L-cysteine), ascorbic acid, insulin or insulin-like factor, hydrocortisone, dexamethasone, bFGF (basic fibroblast growth factor), heparan sulfate, 2-mercaptoethanol, EGF (epidermal growth factor) and an antioxidant.

Meanwhile, in the process of preparing the stem cells to be contained in the stem cell composition according to the present invention, stem cells for intravenous administration may be treated with trypsin in the above-described medium according to the present invention. When cultured stem cells are treated with trypsin, the stem cells can be present as single cells. Herein, trypsin is used to treat cells so as to inhibit the aggregation of the cells to thereby enable the cells to be present as single cells, and any material may be used as a substitute for trypsin, as long as it can inhibit the aggregation of cells.

In addition, in the process of preparing the stem cells to be contained in the stem cell composition according to the present invention, stem cells cultured in the medium according to the present invention may be suspended in an aspirin-containing solution.

As used herein, the term "aspirin-containing solution" refers to a solution containing an aspirin compound. As a solvent for the aspirin-containing solution, physiological saline may preferably be used. In addition to physiological saline, any base that is generally used in the art, such as Hartman-D solution or PBS (phosphate buffered saline), may be used without limitation. Aspirin that is used in the present invention may be a commercially available aspirin formulation or an aspirin-like compound. In an example of the present invention, an aspirin-containing solution was prepared by adding acetylsalicylic acid (Sigma; A5376), Arthalgyl Injection or aspirin lysine (Shin Poong Pharm. Co., Ltd.) to physiological saline. Herein, the content of aspirin added is preferably 0.0001-0.01 mg/ml. If the amount of aspirin added is more than 0.01 mg/ml, the viability of cells will decrease, and if it is less than 0.0001 mg/ml, the effect of inhibiting cell disruption or aggregation will be insufficient.

When stem cells are suspended in the aspirin-containing solution, the disruption and aggregation of the stem cells during transport or storage will not appear, and thus such stem cells are useful in that they can be immediately used for administration into body. Thus, stem cells for intravascular administration are preferably used after they are suspended in aspirin-containing physiological saline.

When cultured stem cells are suspended in the aspirin-containing solution, these cells are useful in that they can be prevented from disruption or aggregation during transport or storage. Thus, stem cells for intravascular administration are preferably used after they are suspended in aspirin-containing physiological saline.

The aspirin-containing solution is a solution containing an aspirin compound. As a solvent for the aspirin-containing solution, physiological saline may preferably be used. In addition to physiological saline, any base that is generally used in the art, such as Hartman-D solution or PBS (phosphate buffered saline), may be used without limitation. Aspirin that is used in the present invention may be a commercially available aspirin formulation or an aspirin-like compound. The amount of aspirin added is preferably 0.0001-0.01 mg/ml. If the amount of aspirin added is more than 0.01 mg/ml, the viability of cells will decrease, and if it is less than 0.0001 mg/ml, the effect of inhibiting cell aggregation will be insufficient.

The stem cell composition for intravenous administration according to the present invention, which contains stem cells prepared according to the above-described method, may be administered intravenously into a patient.

For example, the stem cell composition of the present invention may be administered intravenously to one or more sites (e.g., 2-50 sites) around the target site to be treated, and may be administered at a dose of 1.0×10⁵ to 1.0×10⁸ cells/kg (weight), and preferably 1.0×10⁶ to 1.0×10⁷ cells/kg (weight). However, the dose may vary depending on the patient's weight, age, sex and condition, the dosage form of the composition to be administered, the mode of administration, etc., and can be suitably determined by those skilled in the art.

The frequency of administration of the stem cell composition of the present invention may range from one to several times within clinically acceptable limits of side effects. There may be one or more administration sites. The dose per kg for non-human animals may be the same as that for human, or can be converted from the above-described dose, for example, based on the volume ratio (for example, average value) between the ischemic organs (such as heart) of the human and animal subjects. Animals to be treated according to the present invention include human and other desired mammals, specific examples of which include humans, monkeys, mice, rats, rabbits, sheep, cows and dogs.

The stem cell composition for intravenous administration according to the present invention may contain stem cells at a concentration of 1 X 10⁷ to 5 X 10⁸ cells/ml.

The stem cell composition for intravenous administration according to the present invention may further contain pharmaceutically acceptable carriers and/or additives. For example, the composition may contain sterile water, physiological saline, usual buffer (phosphoric acid, citric acid, or other organic acids), a stabilizer, a salt, am antioxidant (ascorbic acid, etc.), a surfactant, a suspending agent, an isotonic agent or a preservative. For topical administration, the composition of the present invention may be combined with an organic material such as a biopolymer, or an inorganic material such as hydroxyapatite. Specifically, it may be combined with a collagen matrix, a poly(lactic acid) polymer or copolymer, a polyethyleneglycol polymer or copolymer, or a chemical derivative thereof.

The stem cell composition for intravenous administration according to the present invention is preferably prepared as a formulation suitable for injection. For this purpose, the stem cells of the present invention are preferably dissolved in a pharmaceutically acceptable aqueous solution or frozen in a solution. Thus, the stem cell composition for intravenous administration according to the present invention may further contain a pharmaceutically acceptable carrier that can be used to suspend or dilute stem cells. This carrier may be, for example, distilled water, physiological saline, PBS (phosphate buffered saline) or Hartman-D (JW Pharmaceutical Corp., Korea).

In order to provide the composition of the present invention as a pharmaceutical formulation, the composition may contain a carrier or an excipient, and may further contain a stabilizer or an adsorption preventing agent. The formulation may be an injectable formulation, and may contain a pain-relieving agent that can reduce pain upon injection. If necessary, a suitable device may be used.

The stem cell composition for intravenous administration according to the present invention may be may be filled into a syringe, a device, a cryovial in which cells can be frozen, or a pyrogen-free glass vial comprising rubber stoppers and aluminum caps, which contains liquid drugs. As a device for administrating the stem cell composition, a syringe, a multi-syringe or the like may be used. For limb ischemic diseases, a 20-30 gauge needle, which can minimize pain during the administration of cells without causing damage to cells due to the shearing of cells, is used by taking into consideration the depth of a site or muscle to which cells are administered. Also, the syringe or device is made of a material that does not influence cell viability.

The stem cell composition for intravenous administration according to the present invention may, if necessary, contain at least one selected from among suspending agents, solubilizing agents, stabilizers, isotonic agents, preservatives, adsorption-preventing agents, surfactants, diluents, vehicles, pH-adjusting agents, analgesic agents, buffering agents, sulfur-containing reducing agents and antioxidants, depending on the administration mode or formulation thereof.

Examples of the suspending agents may include methylcellulose, Polysorbate 80, hydroxyethylcellulose, gum acacia, gum tragacanth powder, sodium carboxymethylcellulose, polyoxyethylene sorbitan monolaurate, etc. The solubilizing agents include polyoxyethylene hydrogenated castor oil, polysorbate 80, nicotinamide, polyoxyethylene sorbitan monolaurate, Macrogol and castor oil fatty acid ethyl esters. The stabilizers include dextran 40, methylcellulose, gelatin, sodium sulfite, sodium metasulfite, etc. Examples of the isotonic agents are D-mannitol and sorbitol. Examples of the preservatives include methyl parahydroxybenzoate, ethyl parahydroxybenzoate, sorbic acid, phenol, cresol, and chlorocresol. Examples of the adsorption-preventing agents include human serum albumin, lecithin, dextran, ethylene oxide-propylene oxide copolymers, hydroxypropylcellulose, methylcellulose, polyoxyethylene hydrogenated castor oil, and polyethylene glycol. The sulfur-containing reducing agents include N-acetylcysteine, N-acetylhomocysteine, thioctic acid, thiodiglycol, thioethanolamine, thioglycerol, thiosorbitol, thioglycolic acid and salts thereof, sodium thiosulfate, glutathione, and sulfhydryl-containing compounds such as thioalkanoic acid having 1 to 7 carbon atoms. The antioxidants include, for example, erythorbic acid, dibutylhydroxytoluene, butylhydroxyanisole, α-tocopherol, tocopherol acetate, L-ascorbic acid and salts thereof, L-ascorbyl palmitate, L-ascorbyl stearate, sodium bisulfite, sodium sulfite, triamyl gallate, propyl gallate or chelating agents such as disodium ethylenediamine tetraacetate (EDTA), sodium pyrophosphate and sodium metaphosphate. The cryopreservatives include, for example, DMSO, glycerol, etc. Furthermore, the composition of the present invention may comprise conventional additives, such as inorganic salts, including sodium chloride, potassium chloride, calcium chloride, sodium phosphate, potassium phosphate and sodium hydrogen carbonate, and organic salts, including sodium citrate, potassium citrate and sodium acetate.

In an example of the present invention, adipose mesenchymal stem cells were cultured in the medium of the present invention. Adipose mesenchymal stem cells can be obtained in the following manner. First, adipose tissue is isolated from abdominal tissue by liposuction, and then washed with PBS. The washed adipose tissue is cut finely, and then treated with a DMEM medium containing collagenase. The collagenase-treated tissue is washed with PBS, and then centrifuged at 1000 rpm for 5 minutes. The supernatant is removed, and the pellets remaining at the bottom are washed with PBS and centrifuged at 1000 rpm for 5 minutes. The resulting cells are filtered through a 100 *µ*m mesh filter to remove floating material, and then washed with PBS. Then, the cells are cultured in a K-SFM medium containing NAC, ascorbic acid, calcium, EGF, insulin and hydrocortisone while the medium is replaced at 2-day intervals. The cultured mesenchymal stem cells are isolated and subcultured, thereby obtaining adipose mesenchymal stem cells. In addition to this method, any method known in the art may also be used to prepare mesenchymal stem cells.

### EXAMPLES

Hereinafter, the present invention will be described in further detail with reference to examples. It will be obvious to a person having ordinary skill in the art that these examples are illustrative purposes only and are not to be construed to limit or change the scope of the present invention.

### Example 1: Isolation of human adipose tissue-derived mesenchymal stem cells

Adipose tissue was isolated from abdominal tissue by liposuction, and then washed with PBS. The washed adipose tissue was cut finely, and then treated and degraded with a DMEM medium containing collagenase type 1 (1 mg/ml) at 37°C for 2 hours. The collagenase-treated tissue was washed with PBS, and then centrifuged at 1000 rpm for 5 minutes. The supernatant was removed, and the pellets were washed with PBS and centrifuged at 1000 rpm for 5 minutes. The resulting cells were filtered through a 100 *µ*m mesh filter to remove floating material, and then washed with PBS, after which the cells were cultured in a DMEM medium containing 10% FBS, 2 mM NAC (N-acetyl-L-cysteine) and 0.2 mM ascorbic acid.

After overnight, the non-adherent cells were washed out with PBS, and the cells were subcultured to passage 3 in a K-SFM medium containing 5% FBS, 2 mM NAC, 0.2 mM ascorbic acid, 0.09 mM calcium, 5 ng/ml rEGF, 5 *µ*g/ml insulin, 10 ng bFGF, 74 ng/ml hydrocortisone and 1 ng/ml selenium while the medium was replaced at 2-day intervals, thereby obtaining adipose mesenchymal stem cells.

### Example 2: Identification of medium components suitable for culture of stem cells for intravenous administration

Medium 1 containing all FBS, bFGF (basic fibroblast growth factor), insulin, hydrocortisone, EGF (epidermal growth factor), ascorbic acid, NAC (N-acetyl-L-cysteine) and selenium, which are the active ingredients of the K-SFM medium used in Example 1, and media 2 to 10 free of one or more of the active ingredients, were prepared in the following manner.

Medium composition is as follows:
Medium 1: K-SFM medium + FBS + NAC + ascorbic acid + insulin + hydrocortisone + bFGF + EGF + selenium
Medium 2: K-SFM medium + NAC + ascorbic acid + insulin + hydrocortisone + bFGF + EGF + selenium (exclusion of FBS from ingredients of medium 1)
Medium 3: K-SFM medium + FBS + NAC + ascorbic acid + insulin + hydrocortisone + EGF + selenium (exclusion of bFGF from ingredients of medium 1)
Medium 4: K-SFM medium + FBS + NAC + ascorbic acid + hydrocortisone + bFGF + EGF + selenium (exclusion of insulin from ingredients of medium 1)
Medium 5: K-SFM medium + FBS + NAC + ascorbic acid + insulin + bFGF + EGF + selenium (exclusion of hydrocortisone from ingredients of medium 1)
Medium 6: K-SFM medium + FBS + NAC + ascorbic acid + insulin + hydrocortisone + bFGF + selenium (exclusion of EGF from ingredients of medium 1)
Medium 7: K-SFM medium + FBS + NAC + insulin + hydrocortisone + bFGF + EGF + selenium (exclusion of ascorbic acid from ingredients of medium 1)
Medium 8: K-SFM medium + FBS + ascorbic acid + insulin + hydrocortisone + bFGF + EGF + selenium (exclusion of NAC from ingredients of medium 1)
Medium 9: K-SFM medium + FBS + NAC + ascorbic acid + insulin + hydrocortisone + bFGF + EGF (exclusion of selenium from ingredients of medium 1)
Medium 10: K-SFM medium + FBS + NAC + ascorbic acid + insulin + hydrocortisone + selenium (exclusion of bFGF and EGF from ingredients of medium 1).

Adipose stem cells were cultured in each of media 2 to 10 (free of one or more of the active ingredients) and a K-SFM medium (control). The cells were subcultured in each of the media to passage 3, and then treated with trypsin, after which the diameter of the cells was measured with a confocal microscope.

The results of the measurement indicated that the stem cells cultured in the medium used in Example 1 had a size of about 10-15 *µ*m (not shown).

### Example 2: Identification of medium components having effects on the ability of stem cells to proliferate

Adipose stem cells were cultured in media 1 to 10 prepared in Example 2. The adipose stem cells used were isolated from men in their 20s (n=3), 30s (n=3), 70s (n=3) and 80s (n=3). The adipose stem cells prepared according to the method of Example 1 were seeded in each of the media at a concentration of 1 X 10⁵ cells/ml and treated with trypsin at day 1, day 2, day 3 and day 4, after which the number of the cells was counted with a confocal microscope. Table 1 below shows the mean cell count measured for the men (N=3) of each age group. As can be seen in Table 1 below, when the stem cells were cultured to passage 4 according to the culture method of the present invention, stem cells could be prepared at a concentration of about 7 X 10⁵ to 1.1 X 10⁶ cells/ml (see Table 1). In addition, it could be seen that the CPDL (cell population doubling level) was the highest in the case of medium 9, even though it did slightly differ between the age groups. Furthermore, it could be seen that, when 1 X 10⁵ cells/ml of stem cells were seeded, the cell count increased 10-11 times at day 4 in the case of the men in their 20s and 30s, and increased 7-9 times in the case of the men in their 70s and 80s (not shown).

**Table 1: Cell count (cells/ml) of adipose mesenchymal stem cells isolated from each of media 1 to 10 at varying days of culture (the cells count is expressed as mean cell count for each group (N=3)**

| Age | Day (post seeding) | Total Cell Count (x 10⁶/ml) | | | | | | | | | |
|---|---|---|---|---|---|---|---|---|---|---|---|
| | | M1 | M2 | M3 | M4 | M5 | M6 | M7 | M8 | M9 | M10 |
| 20 | Day 1 | 1.8 | 1.6 | 3.9 | 1.8 | 1.5 | 1.6 | 1.5 | 1.5 | 1.0 | 1.3 |
| | Day 2 | 4.0 | 3.2 | 3.6 | 4.4 | 4.0 | 4.7 | 3.9 | 4.4 | 4.0 | 1.2 |
| | Day 3 | 11.2 | 3.0 | 8.9 | 14.0 | 12.9 | 12.8 | 11.8 | 12.8 | 11.2 | 1.3 |
| | Day 4 | 14.6 | 0.8 | 9.9 | 13.7 | 17.1 | 19.2 | 21.8 | 20.7 | 20.2 | 1.7 |
| 30 | Day 1 | 1.8 | 3.1 | 3.7 | 1.7 | 1.7 | 2.2 | 1.7 | 1.4 | 2.3 | 1.5 |
| | Day 2 | 2.7 | 0.8 | 2.2 | 3.2 | 3.0 | 3.2 | 3.2 | 3.4 | 4.4 | 1.2 |
| | Day 3 | 9.0 | 3.0 | 7.1 | 8.9 | 11.8 | 10.5 | 10.6 | 11.8 | 11.5 | 1.3 |
| | Day 4 | 15.9 | 3.0 | 12.1 | 16.2 | 13.2 | 17.0 | 20.5 | 22.6 | 22.1 | 1.6 |
| 70 | Day 1 | 1.4 | 0.8 | 3.4 | 1.3 | 1.3 | 3.4 | 1.2 | 0.9 | 1.4 | 1.1 |
| | Day 2 | 3.1 | 0.8 | 5.1 | 2.3 | 2.6 | 2.3 | 2.8 | 2.4 | 2.8 | 1.2 |
| | Day 3 | 7.3 | 0.8 | 5.6 | 6.9 | 9.7 | 8.3 | 8.1 | 8.2 | 10.4 | 1.3 |
| | Day 4 | 10.5 | 0.6 | 7.4 | 9.4 | 9.2 | 11.0 | 14.7 | 12.9 | 13.1 | 1.2 |
| 80 | Day 1 | 1.7 | 1.1 | 1.7 | 1.6 | 1.3 | 3.3 | 1.3 | 1.3 | 1.4 | 1.2 |
| | Day 2 | 2.7 | 1.1 | 1.9 | 2.2 | 2.7 | 2.8 | 2.8 | 2.9 | 2.1 | 0.8 |
| | Day 3 | 6.4 | 0.8 | 5.8 | 7.3 | 9.9 | 6.1 | 5.9 | 5.7 | 5.6 | 1.1 |
| | Day 4 | 9.9 | 0.7 | 7.1 | 9.9 | 7.8 | 17.0 | 11.3 | 11.4 | 11.0 | 1.1 |

### Example 4: The ability of stem cells to be maintained as single cells

### (1) Treatment with physiological saline containing varying concentrations of acetylsalicylic acid

The adipose mesenchymal stem cells isolated in Example 1 were treated with trypsin, and then suspended at a concentration of 1.0 X 10⁷ cells in physiological saline containing varying concentrations of acetylsalicylic acid (Sigma; A5376), after which the viability of the cells at 12 hours and 24 hours after the suspension was observed. The physiological saline containing acetylsalicylic acid was prepared by adding acetylsalicylic acid to physiological saline at varying concentrations and sonicating it at 37 °C for 30 minutes.

**Table 2**

| | Concentrations of Aspirin | | | |
|---|---|---|---|---|
| Viability | 0mg/10ml | 1.0mg/10ml | 2.0mg/10ml | 5.0mg/10ml |
| 12 hours | 85% | 50% | 20% | 10% |
| 24 hours | 70% | 10% | 10% | 5% |

As a result, it could be seen that, when aspirin was added to 10 ml of physiological saline in an amount of 1.0 mg or more, it was cytotoxic.

### (2) Treatment with physiological saline containing varying concentrations of Arthalgyl Injection

The adipose mesenchymal stem cells isolated in Example 1 were treated with trypsin, and then suspended at a concentration of 1.0 X 10⁷ cells in physiological saline containing varying concentrations of Arthalgyl Injection, after which the viability of the cells at 12 hours and 24 hours after the suspension was observed.

**Table 3**

| | Concentrations of Aspirin | | | |
|---|---|---|---|---|
| Viability | 0mg/10ml | 0.001mg/10ml | 0.01mg/10ml | 0.1mg/10ml |
| 12 hours | 95% | 91% | 90% | 90% |
| 24 hours | 90% | 87% | 85% | 84% |
| 48 hours | 85% | 80% | 77% | 74% |
| 72 hours | 62% | 63% | 61% | 61% |

As a result, it could be seen that, when aspirin was added to 10 ml of physiological saline in an amount of 0.001-0.1 mg, it did not affect the viability of the adipose stem cells. When the adipose stem cells were suspended in the aspirin-containing solution, these cells could be stored for up to 72 hours, but were preferably used for treatment.

### (3) Proliferation ability of stem cells treated with physiological saline containing varying concentrations of Arthalgyl Injection

The adipose mesenchymal stem cells isolated in Example 1 were treated with trypsin, and then suspended at a concentration of 1.0 X 10⁷ cells in physiological saline containing varying concentrations of Arthalgyl Injection, after which the viability of the cells at 24 hours after the suspension was observed.

**Table 4**

| | Concentrations of Aspirin | | | |
|---|---|---|---|---|
| Viability | 0mg/10ml | 0.001mg/10ml | 0.01mg/10ml | 0.1mg/10ml |
| 24 hours | 95% | 92% | 91% | 91% |

As a result, it could be seen that, when Arthalgyl Injection was added to 10 ml of physiological saline in an amount of 0.001-0.1 mg, it did not affect the ability of the adipose stem cells to proliferate.

In addition, the suspended cells were cultured at a concentration of 1.0 X 10⁶ cells for 7 days, and then the cell count and viability of the cells were observed. The results of the observation are shown in Tables 5 and 6 below.

**Table 5: Viability after 24 hours at varying concentrations of aspirin (n=3)**

| | Concentrations of Aspirin | | | |
|---|---|---|---|---|
| Viability | 0mg/10ml | 0.001mg/10ml | 0.01mg/10ml | 0.1mg/10ml |
| Average | 91±1% | 90±0% | 88±2.6% | 87±1% |
| Donor 1 | 90% | 90% | 85% | 87% |
| Donor 2 | 91% | 90% | 89% | 86% |
| Donor 3 | 92% | 90% | 90% | 88% |

**Table 6: Effect of aspirin on cell proliferation during culture after 24 hours of suspension at varying concentrations of aspirin (n=3)**

| | Concentrations of Aspirin | | | |
|---|---|---|---|---|
| Cell count | 0mg/10ml | 0.001mg/10ml | 0.01mg/10ml | 0.1mg/10ml |
| Donor 1 | 8.0 X 10⁶ | 7.6 X 10⁶ | 8.2 X 10⁶ | 7.8 X 10⁶ |
| Donor 2 | 1.18 X 10⁷ | 1.32 X 10⁷ | 1.1 X 10⁷ | 1.12 X 10⁷ |
| Donor 3 | 1.02 X 10⁷ | 1.0 X 10⁷ | 1.3 X 10⁷ | 1.02 X 10⁷ |

### (4) Characteristics of stem cells treated with physiological saline containing varying concentrations of aspirin lysine (Shin Poong Pharm. Co., Ltd, Korea)

The adipose mesenchymal stem cells isolated in Example 1 were treated with trypsin, and then suspended at a concentration of 1.0 X 10⁷ cells in physiological saline containing varying concentrations of aspirin lysine (Shin Poong Pharm. Co., Ltd, Korea). Then, the cells were incubated for 5 days, and the cell count of the cells was measured. In addition, the cells were cold-stored for 24 hours, and then the characteristics of the cells were analyzed by FACS.

**Table 7**

| | Concentrations of Aspirin | |
|---|---|---|
| Expression rate | 0mg/10ml | 0.1mg/10ml |
| CD29 | 99.97% | 99.97% |
| CD31 | 0.00% | 0.21% |
| CD44 | 99.45% | 99.25% |
| CD45 | 0.19% | 0.17% |
| CD90 | 99.73% | 99.66% |
| CD105 | 99.90% | 99.89% |

As a result, it could be seen that the cell viability somewhat decreased depending on the concentration of aspirin lysine, but this decrease was not significant, and that the count of the cells after 5 days of incubation did differ between individuals, but did not almost differ between the concentrations of aspirin lysine. In addition, it was observed that the characteristics of the adipose mesenchymal stem cells, which were suspended in the aspirin-containing physiological saline for 24 hours and then cold-stored for 24 hours, were not dependent on the concentration of aspirin lysine.

### INDUSTRIAL APPLICABILITY

The stem cell composition for intravenous administration according to the present invention can deliver stem cells to an injury site without causing vascular occlusion after intravenous administration, and thus exhibit excellent therapeutic effects. Accordingly, the stem cell composition of the present invention can significantly increase the therapeutic effects of stem cells after intravascular administration.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims and equivalents thereof.

## Claims

1. A stem cell composition for intravenous administration, which contains stem cells at a concentration of 1 X 10⁷ to 5 X 10⁸ cells/ml, in which the stem cells have a diameter of 10-20 *µ*m and are present as single cells.

2. The stem cell composition of claim 1, wherein the stem cells have a diameter of 10-15 *µ*m.

3. The stem cell composition of claim 1, wherein the stem cells contained in the stem cell composition for intravenous administration are prepared by the culture thereof in a medium containing a basal medium; and at least two components selected from the group consisting of N-acetyl-L-cysteine (NAC), ascorbic acid, insulin or insulin-like factor, hydrocortisone, dexamethasone, bFGF (basic fibroblast growth factor), heparan sulfate, 2-mercaptoethanol, EGF (epidermal growth factor), and antioxidant.

4. The stem cell composition of claim 3, wherein the basal medium is selected from the group consisting of M199/F12(mixture)(GIBCO), MEM-alpha medium (GIBCO), low-concentration glucose-containing DMEM medium (Welgene), MCDB 131 medium (Welgene), IMEM medium(GIBCO), K-SFM, DMEM/F12 medium, PCM medium, and MSC expansion medium (Chemicon).

5. The stem cell composition of claim 3, wherein the antioxidant is selected from the group consisting of selenium, ascorbic acid, vitamin E, catechin, lycopene, beta-carotene, coenzyme Q-10, EPA (eicosapentaenoic acid), and DHA (docosahexanoic acid).

6. The stem cell composition of claim 5, wherein the antioxidant is selenium.

7. The stem cell composition of claim 3, wherein the medium additionally contains FBS (fetal bovine serum) or calcium.

8. The stem cell composition of claim 3, wherein the cultured stem cells are treated with trypsin.

9. The stem cell composition of claim 3 or 8, wherein the cultured stem cells are suspended in an aspirin-containing solution.

10. The stem cell composition of claim 9, wherein the aspirin-containing solution additionally contains a solution selected from the group consisting of physiological saline, Hartman-D solution, and PBS (phosphate buffered saline).

11. The stem cell composition of claim 9, wherein the aspirin is isosorbide-based aspirin or nicotinic acid-based aspirin.

12. The stem cell composition of claim 9, wherein the content of aspirin added is 0.0001-0.01 mg/ml.

13. The stem cell composition of claim 1, wherein the stem cells are adult stem cells.

14. The stem cell composition of claim 13, wherein the stem cells are adipose tissue-derived mesenchymal stem cells.

15. A method of preparing a stem cell composition for intravenous administration, which contains stem cells at a concentration of 1 X 10⁷ to 5 X 10⁸ cells/ml, in which the stem cells have a diameter of 10-20 *µ*m and are present as single cells, the method comprising the steps of:
(a) culturing adipose tissue- or epithelial tissue-derived adult stem cells in a medium containing a basal medium; and at least two components selected from the group consisting of N-acetyl-L-cysteine (NAC), ascorbic acid, insulin or insulin-like factor, hydrocortisone, dexamethasone, bFGF (basic fibroblast growth factor), heparan sulfate, 2-mercaptoethanol, EGF (epidermal growth factor), and antioxidant;
(b) treating the stem cells cultured in step (a) with trypsin;
(c) suspending the stem cells cultured in step (a) or the stem cells treated with trypsin in step (b) in an aspirin-containing solution; and
(d) preparing the stem cell composition for intravenous administration, which contains the stem cells obtained in step (c) at a concentration of 1 X 10⁷ to 5 X 10⁸ cells/ml.
